# EUROPEAN PATENT SPECIFICATION

(11) **EP 3 535 021 B1**
(45) Date of publication and mention of the grant of the patent: **22.06.2022**
(21) Application number: 17868179.7
(22) Date of filing: 06.11.2017
(51) Int. Cl.: A61N 1/36

(54) **FUNCTIONAL ELECTRICAL STIMULATION ERGOMETER INCLUDING AUTOMATIC SPASM CONTROL**
FUNKTIONELLES ELEKTRISCHES STIMULATIONSERGOMETER MIT AUTOMATISCHER SPASMUSSTEUERUNG
ERGOMÈTRE À STIMULATION ÉLECTRIQUE FONCTIONNELLE COMPRENANT UN RÉGLAGE AUTOMATIQUE DES SPASMES

(30) Priority: 04.11.2016 US 201662417477 P
(43) Date of publication of application: 11.09.2019
(73) Proprietor: Restorative Therapies, Baltimore, Maryland 21231 (US)
(72) Inventor: DUNCAN, Michael, Lane Cove, New South Wales 2066 (AU)
(74) Representative: Barker Brettell LLP
(86) International application number: PCT/US2017/060184
(87) International publication number: WO 2018/085770

(56) References cited:
- JP-A- 2009 039 454
- US-A- 4 691 694
- US-A- 4 691 694
- US-A- 6 010 468
- US-A1- 2004 023 759
- US-A1- 2004 023 759
- US-A1- 2004 122 483
- US-A1- 2007 208 392
- US-A1- 2010 331 603
- US-A1- 2013 053 734
- US-A1- 2013 053 734
- US-A1- 2013 053 736
- US-A1- 2013 158 622
- US-A1- 2013 218 247
- US-A1- 2015 148 866
- US-A1- 2016 016 036

## Description

### Field of the Invention

The invention relates to functional electrical stimulation (FES) systems for rehabilitation of individuals suffering from neurological injuries. More particularly, the present invention relates to a device that utilizes both mechanical and electrical stimulation of individual's muscles.

### Background of the Invention

Spinal cord injured patients gain great benefit from riding on FES enabled ergometers. However, when a patient's limbs are set in motion and/or when stimulation is applied at the start of a session many patients suffer from muscle spasms. This spasm often prevents the patients from riding for several minutes until the spasms have subsided. This can cause time to be lost by both the patient and therapist / care-giver and results in reducing the benefits that the patient receives from the FES ergometer.

Current techniques to minimize spasm include:
1, Stretching the patient's legs prior to riding. The problem with this approach is that it is labor intensive and does not always achieve the desired results.
2. Having the therapist observe the patient as stimulation is being applied and manually prevent the stimulation from increasing when the patient is showing early signs of spasm. The problem with this approach is that it requires a well-trained therapist to be present when the patient cycles. This is not always practical.
3. Setting the FES ergometer to increase the stimulation levels on the subject very slowly so that the patient's muscles accommodate the stimulation. The problem with this solution is that a therapist needs to be trained to know how to set-up the FES ergometer for a patient known to have spasms. Another problem is that it takes longer for the patient to receive the maximum benefit of FES cycling because the stimulation ramps up slowly. The patient may only occasionally suffer from spasm in which case it would be better to ramp up stimulation more quickly when the patient is not suffering from spasm.

US2013/053734 discloses therapeutic exercise devices with extremity supports each equipped with a servo motor driven by a primary motor and a functional electrical stimulation unit.

### Summary of the Invention

According to an embodiment of the invention, there is provided a functional electrical stimulation device having a left extremity support and a right extremity support, wherein both extremity supports are connected to a primary drive motor that causes the extremity supports to move in a reciprocal motion; the left extremity support is connected to a left extremity support servo motor and the right extremity support is connected to a right extremity support servo motor, wherein each of said right and left extremity support servo motors causes its respective extremity support to rotate about an axis. The device further has a control unit connected to the left and right extremity support servo motors which controls the actuation of the left and right extremity support servo motors. The control unit also includes functional electrical stimulation leads attached to skin adhesive electrodes which deliver electrical stimulation to a patient's muscles.

The device also includes an automatic spasm control system including a monitor which continuously monitors the torque required to rotate one or both of the left and right extremity supports and a controller configured to calculate a rate of change of said torque, and to calculate a maximum electrical stimulation, a maximum stimulation ramp-up rate, a maximum servo-motor speed and a maximum servo-motor ramp-up rate.

According to further embodiments, the functional electrical stimulation system has two drive arms connecting the primary drive motor to each of said left and right extremity supports, the drive arms optionally having various attachment points for connecting to corresponding right and left cranks at different attachment positions. According to further embodiments, the extremity supports are attached to the drive aims by the corresponding left and right cranks.

According to further embodiments of the invention, the controller of said automatic spasm control system is configured to identify a possible onset of spasm when a monitored amount of torque or rate of change of torque exceeds a predetermined value, and when a monitored amount of torque or rate of change of torque exceeds said predetermined value, to compare a current level of electrical stimulation to said calculated maximum stimulation and to compare a current crank speed to said calculated maximum crank speed and to send instructions to said control unit connected to the left and right extremity support servo motors and to said functional electrical stimulation leads to reduce the amount of electrical stimulation and crank speed.

According to further embodiments of the invention, there is provided a therapeutic muscle exercise device having a cycling base configured to accept a subject's upper or lower extremities and assist a subject in exercising the subject's muscles; the cycling base having a first extremity support and a second extremity support, wherein said extremity supports are driven or resisted by a motor, the device further including a functional electrical stimulation controller configured to control the motor, wherein the functional electrical stimulation controller and the first and second extremity supports and cycling base are configured to assist a subject in moving his or her upper or lower extremities; said device further including an automatic spasm control system comprising a monitor configured to continuously monitor a torque required to rotate one or both of said left and right extremity supports, said automatic spasm control system configured to calculate a rate of change of said torque, a maximum crank speed and a maximum crank ramp-up rate, a maximum electrical stimulation, a maximum stimulation ramp-up rate.

According to further embodiments of the invention the functional electrical stimulation controller manages isometric functional electrical stimulation to the subject's muscles.

### Description of the Drawings

The subsequent description of the preferred embodiments of the present invention refers to the attached drawings, wherein:

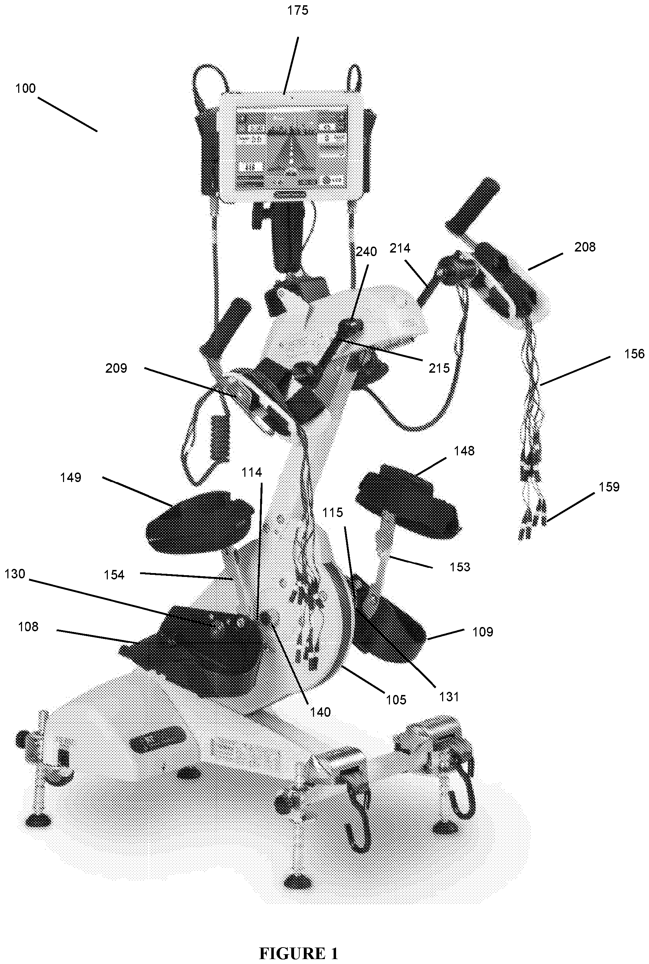

Figure 1 shows an RT300TM Functional Electrical Stimulation cycling system by Restorative Therapies, with an automatic spasm control system of the invention installed in the RT300TM's controller.

### Detailed Description of the Invention

The invention summarized above may be better understood by referring to the following description, the accompanying drawings, and the claims listed below. This description of an embodiment, set out below to enable one to practice an implementation of the invention, is not intended to limit the preferred embodiment, but to serve as a particular example thereof. While the invention is described herein in the context of an RT300-SLSA cycling system, it may be used in conjunction with any FES system, including the systems described in U.S. Patent Nos. 8,905,951 and 9,511,256.

As shown in FIG. 1, one embodiment of the present invention comprises a therapeutic muscle exercise device 100 used to treat patients who may have partial or entire paralysis of the arms and/or legs. The device 100 comprises a cycling base 105 that is mounted on a mobile support 103.

The cycling base 105 has a first extremity support 108 and a second extremity support 109. It is contemplated that in some embodiments a single extremity support may be included to facilitate treatment of subjects who have lost a limb. Each extremity support 108 and 109 are attached to the cycling base by a first rotating arm 114 and a second rotating arm 115, respectively.

The rotating arms 114 and 115 attach to the extremity supports 108 and 109 at a extremity support pivot point 130 and 131. The configuration allows the foot of the subject to rotate as normally as it would when performing a cycling motion naturally. The rotating arms connect to the cycling base 105 at a base pivot point 140. In a preferred embodiment, the base pivot point 140 comprises a shaft that passes through a channel that extends through the base; the shaft connects the base end of the first rotating arm 114 to the base end of the second rotating arm 115. In such embodiment, the shaft then rotates uniformly around the base pivot point 140. In other embodiments, the rotating arms 114, 115 may connect to two independently rotatable shafts at the base pivot point 140 allowing the extremity supports 108 and 109 to rotate independently of one-another.

The cycling base 105 also includes two calf supports 148, 149. In some embodiments, the calf supports 148 and 149 are connected to the extremity supports 108 and 109 though an attachment link 153, 154. In other embodiments, the calf supports 148 and 149 may not be linked to the extremity supports 108 and 109.

The device 100 also includes a functional electrical stimulation controller 175. The controller 175 manages the electrical impulses provided to the subject's muscles through a series of leads 159, which are connected to the controller 175 through a lead cable 156. The controller 175 also controls a motor (not shown) that controls the speed of rotation of the rotating arms 114 and 115. The controller 175 coordinates the rotation of the rotating arms 114 and 115 in conjunction with functional electrical stimulation through the leads 159 depending on the level of need of the subject. In some instances, the controller 175 will direct full movement of the rotating arms 114 and 115 in conjunction with electrical stimulation resulting in fully FES evoked movement. In the alternative, the controller 175 may reduce the level of assistance provided by the motor and the electrical stimulation allowing volitional movement by the subject. In yet a further embodiment, the controller 175 will not provide any assistance in order to allow the subject to rotate the extremity supports 108, 109 without assistance. The controller 175 provides such assistance as needed by the subject and directed by the user.

Likewise, wrist and hand supports 208 and 209 may be provided and configured to attach to rotating arms 214 and 215 at base pivot point 240, and subsequent attachment to wrist and hand supports 208 and 209.

In an example not according to the invention and present for illustration purposes only, a method for exercising a subjects extremities is provided. The system continuously rotates at the set speed and it either assists or resists the patients efforts. The patient's efforts are either volitional or evoked by FES. In a first step of the method the system's motor moves the subject's extremities and the controller provides initial muscle electrical stimulation. This first step can be referred to as a warm up step. In a subsequent step, referred to as active transition, the controller increases muscle electrical stimulation and reduces motor assistance until the muscle takes over movement of the extremity supports or 100% stimulation threshold is reached. In a subsequent step, referred to as the active step, electrical stimulation evokes coordinated muscle contractions as the rotating arms 114 and 115 (and/or 214 and 215) turn around the pivot point 140 (and/or 240) resulting in the subjects muscles performing the cycling motion. The combination of the rotating arms 114 and 115 (and/or 214 and 215) connected at the pivot point 140 (and/or 240) is also referred to herein as the crank. Once muscle fatigue is reached and/or detected by the controller, electrical stimulation is reduced and the motor of the cycling base takes over for cycling motion. In a final step, referred to as cool down, the motor completely takes over cycling motion allowing subject's muscles to rest while permitting movement through the range of motion.

According to a further embodiment, an automatic spasm control feature of the present invention monitors the amount of torque required to move the patient's arms or legs from the beginning of the therapy session. The system also monitors the pattern and rate of change, or smoothness, of the required torque through each rotation of the ergometer crank. The automatic spasm control system of the invention uses increases in required torque as well as decreases in the smoothness of the required torque to detect muscle tone and to detect if a muscle spasm is occurring.

For each amount of muscle tone detected, the automatic spasm control system of the invention sets a maximum stimulation level, a maximum stimulation ramp-up rate, a maximum crank speed, and a crank ramp-up rate. If a full muscle spasm occurs, then the automatic spasm control system inhibits the motor from rotating the crank for a period of time, and then tries to start the therapy session again.

The following example follows a patient starting a leg therapy session and the stimulation level is increasing. If the patient experiences the onset of a spasm, the torque required to assist leg rotation will begin to fluctuate which fluctuation in torque will be detected by the automatic spasm control system. Similarly, if the patient starts to exhibit high muscle tone, the motor torque required to assist the leg crank rotation will increase and the automatic spasm control system will detect this increase. In either event, automatic spasm control system will calculate the maximum stimulation level and cycling cadence appropriate to prevent the muscle tone from increasing or developing into a muscle spasm.

If the current therapy is stimulating at a higher level than the calculated maximum stimulation level appropriate to prevent the muscle tone from increasing or developing into a spasm, the automatic spasm control system will cause the stimulation level to be decreased accordingly. Likewise, if the current leg therapy cadence is currently going faster than the maximum cycling cadence to prevent the muscle tone from increasing or developing into a spasm, the automatic spasm control system will cause the cadence rate to be reduced.

Conversely, if the current stimulation level is lower than the calculated maximum stimulation value, then the system will calculate the maximum stimulation level ramp up rate that is appropriate to prevent the muscle tone from increasing or a muscle spasm from developing. The system will allow the stimulation level to increase at a rate no greater than this maximum.

If the cadence is lower than the calculated maximum cycling cadence value, then the system will calculate the maximum speed ramp up rate that is appropriate to prevent the muscle tone from increasing or a muscle spasm from occurring. The system will allow the cadence to increase at a rate no greater than this calculated maximum rate.

If the muscle tone continues to increase, then all of these maximum levels will be further reduced. This acts to allow the patient's muscles to accommodate the stimulation so that eventually the muscle tone will reduce. As the muscle tone reduces, the system will increase all these maximum levels. Once there is no or minimal muscle tone, no limits are imposed by the system. At this point the FES therapy session will continue using its preset stimulation level and cadence parameters.

In the event that the detected muscle tone does not decrease over time, indicating that the muscles are either not accommodating the prescribed cadence or stimulation levels, the automatic spasm control system will slowly allow a higher cadence and stimulation level. This allows a patient to progress to active cycling even though he/she is exhibiting tone. In this case it is likely that the detected tone was caused not by spasm, but by another factor such as myotatic reflex. According to various different embodiments of the invention, the system may make adjustments to cadence and stimulation levels appropriately for spasm and myotatic reflex.

## Claims

1. A functional electrical stimulation device comprising:
a left extremity support (108) and a right extremity support (109), wherein both extremity supports are connected to a primary drive motor that is configured to move the extremity supports in a reciprocal motion, said left extremity support connected to a left extremity support servo motor and said right extremity support connected to a right extremity support servo motor, wherein each of said right and left extremity support servo motors is configured to rotate its respective extremity support about an axis;
a control unit (175) connected to the left and right extremity support servo motors and which controls the actuation of the left and right extremity support servo motors, wherein the control unit comprises functional electrical stimulation leads attached to skin adhesive electrodes and wherein said adhesive electrodes are configured to deliver electrical stimulation to a patient's muscles; and
**characterised in that** said device further comprises an automatic spasm control system comprising a monitor and a controller, the monitor configured to continuously monitor a torque required to rotate one or both of said left and right extremity supports, said controller configured to calculate a rate of change of said torque, a maximum electrical stimulation, a maximum stimulation ramp-up rate, a maximum servo motor speed and a maximum servo motor ramp-up rate.

2. The functional electrical stimulation device of claim 1, further comprising two drive arms (114;115) connecting said primary drive motor to each of said left and right extremity supports (108; 109), wherein said drive arms comprise various attachment points for connecting to corresponding right and left cranks at different attachment positions.

3. The functional electrical stimulation device of claim 2, wherein the extremity supports (108; 109) are attached to the drive arms (114;115) by the corresponding left and right cranks.

4. The functional electrical stimulation device of claim 1, said controller of said automatic spasm control system further configured to identify a possible onset of spasm when a monitored amount of torque or rate of change of torque exceeds a predetermined value, and, when the monitored amount of torque or rate of change of torque exceeds said predetermined value, said controller is configured to compare a current level of electrical stimulation to said calculated maximum electrical stimulation and to compare a current crank speed to said calculated maximum crank speed and to send instructions to said control unit connected to the left and right extremity support servo motors and to said functional electrical stimulation leads to reduce the amount of electrical stimulation and the crank speed.

5. A therapeutic muscle exercise device, comprising:
a cycling base (105) configured to accept a subject's upper or lower extremities and assist a subject in exercising the subject's muscles, the cycling base comprising a first extremity support (108) and a second extremity support (109), wherein said extremity supports are driven or resisted by a motor ;
a functional electrical stimulation controller (175) configured to control the motor, wherein the functional electrical stimulation controller and the first and second extremity supports and cycling base are configured to assist a subject in moving his or her upper or lower extremities; and
**characterised by** an automatic spasm control system comprising a monitor configured to continuously monitor a torque required to rotate one or both of said left and right extremity supports, said automatic spasm control system configured to calculate a rate of change of said torque, a maximum crank speed, a maximum crank ramp-up rate, a maximum electrical stimulation, and a maximum stimulation ramp-up rate.

6. The device of claim 1, wherein the functional electrical stimulation controller (175) is configured to control isometric functional electrical stimulation to the subject's muscles.

7. The device of claim 5, said automatic spasm control system configured to identify a possible onset of spasm when a monitored amount of torque or rate of change of torque exceeds a predetermined value, and, when a monitored amount of torque or rate of change of torque exceeds said predetermined value, to compare a current crank speed to said calculated maximum crank speed and to compare a current level of electrical stimulation to said calculated maximum electrical stimulation.

## Patentansprüche

1. Funktionelle elektrische Stimulationsvorrichtung, umfassend:
eine linke Extremitätsstütze (108) und eine rechte Extremitätsstütze (109), wobei beide Extremitätsstützen mit einem Primärantriebsmotor verbunden sind, der ausgestaltet ist, um die Extremitätsstützen in einer reziproken Bewegung zu bewegen, wobei die linke Extremitätsstütze mit einem Servo-motor der linken Extremitätsstütze verbunden ist und die rechte Extremitätsstütze mit einem Servo-motor der rechten Extremitätsstütze verbunden ist, wobei jeder der Servomotoren für die rechte und linke Extremitätsstütze ausgestaltet ist, um seine jeweilige Extremitätsstütze um eine Achse zu rotieren;
eine Steuereinheit (175), die mit den Servomotoren der linken und rechten Extremitätsstütze verbunden ist und die Betätigung der Servomotoren der linken und rechten Extremitätsstütze steuert, wobei die Steuereinheit funktionelle elektrische Stimulationszuleitungen umfasst, die an Hautklebeelektroden befestigt sind, und wobei die Klebeelektroden ausgestaltet sind, um elektrische Stimulation an die Muskeln eines Patienten abzugeben; und
**dadurch gekennzeichnet, dass** die Vorrichtung des Weiteren ein automatisches Spasmussteuersystem umfasst, das eine Überwachung und eine Steuerung umfasst, wobei die Überwachung ausgestaltet ist, um kontinuierlich ein Drehmoment zu überwachen, das erforderlich ist, um eine oder beide der linken und rechten Extremitätsstützen zu rotieren, wobei die Steuerung ausgestaltet ist, um eine Änderungsrate des Drehmoments, eine maximale elektrische Stimulation, eine maximale Stimulationshochfahrrate, eine maximale Servomotordrehzahl und eine maximale Servomotorhochfahrrate zu berechnen.

2. Funktionelle elektrische Stimulationsvorrichtung nach Anspruch 1, des Weiteren umfassend zwei Antriebsarme (114; 115), die den Primärantriebsmotor mit jeder der linken und rechten Extremitätsstützen (108; 109) verbinden, wobei die Antriebsarme verschiedene Befestigungspunkte zum Verbinden mit entsprechenden rechten und linken Kurbeln an unterschiedlichen Befestigungspositionen umfassen.

3. Funktionelle elektrische Stimulationsvorrichtung nach Anspruch 2, wobei die Extremitätsstützen (108; 109) mittels der entsprechenden linken und rechten Kurbel an den Antriebsarmen (114; 115) befestigt sind.

4. Funktionelle elektrische Stimulationsvorrichtung nach Anspruch 1, wobei die Steuerung des automatischen Spasmussteuersystems des Weiteren ausgestaltet ist, um einen möglichen Beginn eines Spasmus zu identifizieren, wenn ein überwachter Betrag des Drehmoments oder der Änderungsrate des Drehmoments einen vorgegebenen Wert überschreitet, und wobei, wenn der überwachte Betrag des Drehmoments oder der Änderungsrate des Drehmoments den vorgegebenen Wert überschreitet, die Steuerung ausgestaltet ist, um ein aktuelles Niveau der elektrischen Stimulation mit der berechneten maximalen elektrischen Stimulation zu vergleichen und eine aktuelle Kurbeldrehzahl mit der berechneten maximalen Kurbeldrehzahl zu vergleichen und Anweisungen an die Steuereinheit zu senden, die mit den Servomotoren der linken und rechten Extremitätsstütze und den funktionellen elektrischen Stimulationszuleitungen verbunden ist, um den Betrag der elektrischen Stimulation und der Kurbeldrehzahl zu reduzieren.

5. Therapeutische Muskelübungsvorrichtung, umfassend:
eine Pedalierbasis (105), die ausgestaltet ist, um die oberen oder unteren Extremitäten eines Subjekts aufzunehmen und das Subjekt bei der Übung der Muskeln des Subjekts zu unterstützen, wobei die Pedalierbasis eine erste Extremitätsstütze (108) und eine zweite Extremitätsstütze (109) umfasst, wobei die Extremitätsstützen durch einen Motor angetrieben werden oder der Motor diesen Widerstandskraft verleiht;
eine funktionelle elektrische Stimulationssteuerung (175), die ausgestaltet ist, um den Motor zu steuern, wobei die funktionelle elektrische Stimulationssteuerung und die erste und zweite Extremitätsstütze und die Pedalierbasis ausgestaltet sind, um das Subjekt bei der Bewegung seiner bzw. ihrer oberen oder unteren Extremitäten zu unterstützen; und
**gekennzeichnet durch**
ein automatisches Spasmussteuersystem, das eine Überwachung umfasst, die ausgestaltet ist, um kontinuierlich ein Drehmoment zu überwachen, das erforderlich ist, um eine oder beide der linken und rechten Extremitätsstützen zu rotieren, wobei das automatische Spasmussteuersystem ausgestaltet ist, um eine Änderungsrate des Drehmoments, eine maximale Kurbeldrehzahl, eine maximale Kurbelhochfahrrate, eine maximale elektrische Stimulation und eine maximale Stimulationshochfahrrate zu berechnen.

6. Vorrichtung nach Anspruch 1, wobei die funktionelle elektrische Stimulationssteuerung (175) ausgestaltet ist, um isometrische funktionelle elektrische Stimulation der Muskeln des Subjekts zu steuern.

7. Vorrichtung nach Anspruch 5, wobei das automatische Spasmussteuersystem ausgestaltet ist, um einen möglichen Beginn des Spasmus zu identifizieren, wenn ein überwachter Betrag an Drehmoment oder Änderungsrate des Drehmoments einen vorgegebenen Wert überschreitet, und wenn ein überwachter Betrag an Drehmoment oder Änderungsrate des Drehmoments den vorgegebenen Wert überschreitet, eine aktuelle Kurbeldrehzahl mit der berechneten maximalen Kurbeldrehzahl zu vergleichen und das aktuelle Niveau der elektrische Stimulation mit der berechneten maximalen elektrischen Stimulation zu vergleichen.

## Revendications

1. Dispositif de stimulation électrique fonctionnelle comprenant :
un support d'extrémité gauche (108) et un support d'extrémité droite (109), les deux supports d'extrémité étant reliés à un moteur d'entraînement primaire qui est configuré pour déplacer les supports d'extrémité dans un mouvement de va-et-vient, ledit support d'extrémité gauche étant relié à un servomoteur de support d'extrémité gauche et ledit support d'extrémité droite étant relié à un servomoteur de support d'extrémité droite, chacun desdits servomoteurs de support d'extrémité droite et gauche étant configuré pour faire tourner son support d'extrémité respectif autour d'un axe ;
une unité de commande (175) reliée aux servomoteurs de support d'extrémité gauche et droite et qui commande l'actionnement des servomoteurs de support d'extrémité gauche et droite, l'unité de commande comprenant des fils de stimulation électrique fonctionnelle fixés à des électrodes adhésives de peau et lesdites électrodes adhésives étant configurées pour délivrer une stimulation électrique aux muscles d'un patient ; et
**caractérisé en ce que** ledit dispositif comprend en outre un système de réglage automatique des spasmes comprenant un moniteur et un dispositif de commande, le moniteur étant configuré pour surveiller en continu un couple requis pour faire tourner l'un ou les deux supports d'extrémité gauche et droite, ledit dispositif de commande étant configuré pour calculer un taux de changement dudit couple, une stimulation électrique maximale, une vitesse de montée en puissance de stimulation maximale, une vitesse de servomoteur maximale et une vitesse de montée en puissance de servomoteur maximale.

2. Dispositif de stimulation électrique fonctionnelle selon la revendication 1, comprenant en outre deux bras d'entraînement (114 ; 115) reliant ledit moteur d'entraînement primaire à chacun desdits supports d'extrémité gauche et droite (108 ; 109), lesdits bras d'entraînement comprenant divers points de fixation pour se relier à des manivelles droite et gauche correspondantes à différentes positions de fixation.

3. Dispositif de stimulation électrique fonctionnelle selon la revendication 2, les supports d'extrémité (108 ; 109) étant fixés aux bras d'entraînement (114 ; 115) par les manivelles gauche et droite correspondantes.

4. Dispositif de stimulation électrique fonctionnelle selon la revendication 1, ledit dispositif de commande dudit système de réglage automatique des spasmes étant en outre configuré pour identifier un début possible de spasme lorsqu'une quantité surveillée de couple ou de taux de changement de couple dépasse une valeur prédéterminée, et, lorsque la quantité surveillée de couple ou de taux de changement de couple dépasse ladite valeur prédéterminée, ledit dispositif de commande étant configuré pour comparer un niveau actuel de stimulation électrique à ladite stimulation électrique maximale calculée et pour comparer une vitesse de manivelle actuelle à ladite vitesse de manivelle maximale calculée et pour envoyer des instructions à ladite unité de commande reliée aux servomoteurs de support des extrémités gauche et droite et auxdits fils de stimulation électrique fonctionnelle pour réduire la quantité de stimulation électrique et la vitesse de manivelle.

5. Dispositif d'exercice musculaire thérapeutique, comprenant :
une base de cyclisme (105) configurée pour accepter les extrémités supérieures ou inférieures d'un sujet et aider un sujet à exercer ses muscles, la base de cyclisme comprenant un premier support d'extrémité (108) et un second support d'extrémité (109), lesdits supports d'extrémité étant entraînés ou résistés par un moteur ;
un dispositif de commande de stimulation électrique fonctionnelle (175) configuré pour commander le moteur, le dispositif de commande de stimulation électrique fonctionnelle et les premier et second supports d'extrémité et la base de cyclisme étant configurés pour aider un sujet à déplacer ses extrémités supérieures ou inférieures ; et
**caractérisé par** un système de réglage automatique des spasmes comprenant un moniteur configuré pour surveiller en continu un couple requis pour faire tourner l'un ou les deux supports d'extrémité gauche et droite, ledit système de réglage automatique des spasmes étant configuré pour calculer un taux de changement dudit couple, une vitesse maximale de la manivelle, une vitesse maximale de montée en puissance de la manivelle, une stimulation électrique maximale et une vitesse maximale de montée en puissance de la stimulation.

6. Dispositif selon la revendication 1, le dispositif de commande de stimulation électrique fonctionnelle (175) étant configuré pour commander une stimulation électrique fonctionnelle isométrique aux muscles du sujet.

7. Dispositif selon la revendication 5, ledit système de réglage automatique des spasmes étant configuré pour identifier un début possible de spasme lorsqu'une quantité de couple ou un taux de changement de couple surveillé dépasse une valeur prédéterminée, et, lorsqu'une quantité de couple ou un taux de changement de couple surveillé dépasse ladite valeur prédéterminée, pour comparer une vitesse de manivelle actuelle à ladite vitesse de manivelle maximale calculée et pour comparer un niveau actuel de stimulation électrique à ladite stimulation électrique maximale calculée.
